(19) Europäisches Patentamt
European Patent Office
Office européen des brevets

(11) Veröffentlichungsnummer: **0 154 122**
**B1**

(12) # EUROPÄISCHE PATENTSCHRIFT

(45) Veröffentlichungstag der Patentschrift: **20.04.88**

(51) Int. Cl.⁴: **C 07 D 239/30**

(21) Anmeldenummer: **85100335.0**

(22) Anmeldetag: **15.01.85**

(54) **Verfahren zur selektiven Hydrierung von Chlor enthaltenden Pyrimidinen und neue Pyrimidine.**

(30) Priorität: **24.01.84 DE 3402194**

(43) Veröffentlichungstag der Anmeldung:
**11.09.85 Patentblatt 85/37**

(45) Bekanntmachung des Hinweises auf die Patenterteilung:
**20.04.88 Patentblatt 88/16**

(84) Benannte Vertragsstaaten:
**BE CH DE FR GB IT LI**

(56) Entgegenhaltungen:
**CHEMISCHE BERICHTE, 110. Jahrgang Nr. 6, 1977, Weinheim M. PROSTENIK und I. BUTULA, "Selektive Reduktion einiger halogen-substituierten Pyridine der Vitamin B-6-Reihe" Seiten 2106-2113**
**JOURNAL OF THE CHEMICAL SOCIETY, Sektion C, 1969, London R.E. BANKS et al., "Heterocyclic Polyfluoro-compounds" Seiten 1866-1867**
**AUSTRALIAN JOURNAL OF CHEMISTRY, Band 21, 1968, Melbourne D.J. BROWN und T.-C. LEE, "Pyrimidine Reactions" Seiten 243-255**
**JOURNAL OF FLUORINE CHEMISTRY, Band 21, 1982, Lausanne E. KLAUKE et al., "Fluorinated Heterocyclic Compounds" Seiten 495-513**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Baasner, Bernd. Dr., Hamberger Strasse 27d, D-5090 Leverkusen 3 (DE)**
Erfinder: **Klauke, Erich. Dr., Eichendorffweg 8, D-5068 Odenthal (DE)**
Erfinder: **Schündehütte, Karl-Heinz. Prof. Dr., Klief 75, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 154 122 B1

ACTORUM AG

## Beschreibung

Die vorliegende Erfindung betrifft ein Verfahren zum teilweisen oder vollständigen Entchlorieren von Fluor und Chlor enthaltenden Pyrimidinen, d.h. zum Ersetzen von in Pyrimidinen enthaltenem Chlor durch Wasserstoff ohne nennenswerten Angriff auf sonstige Substituenten und/oder den aromatischen Ring. Die vorliegende Erfindung betrifft weiterhin neue Pyrimidine, die auf diese Weise zugänglich sind. Beide Gegenstände sind unten näher definiert.

Es ist bekannt, dass man Tetrafluorpyrimidin mit Lithiumaluminiumhydrid in Ether zu einem Gemisch von 2,4,5-Trifluor- und 2,5-Difluorpyrimidin hydrieren kann (siehe J. Chem. Soc. (C), 1969, Seiten 1866–1867). Hierbei ist der Umsatz jedoch nicht vollständig und die beiden Produkte fallen unselektiv an, d.h. stets im Gemisch miteinander. Ausserdem ist dieses Verfahren wegen des Einsatzes des schwierig zu handhabenden Lithiumaluminiumhydrids für eine Ausübung im technischen Massstab nicht geeignet.

Es ist auch bekannt, dass man 2,4,6-Trichlor-5-methylpyridin mit Zink-Staub in Benzol in Gegenwart von konzentrierter Ammoniaklösung reduzieren kann (siehe Austral. J. Chem. 21, 243–245 (1968) ). Auch bei diesem Verfahren fallen mehrere Produkte im Gemisch miteinander an.

Als technologischer Hintergrund für die vorliegende Erfindung kommt noch Chem. Ber. 110, 2106–2113 (1977) in Betracht.

Es wurde nun ein Verfahren zum teilweisen oder vollständigen Entchlorieren von Fluor und Chlor enthaltenden Pyrimidinen gefunden, das dadurch gekennzeichnet ist, dass man Fluor und Chlor enthaltende Pyrimidine der Formel (I)

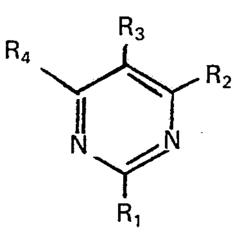

(I),

in der

$R_1$, $R_2$, $R_3$ und $R_4$ gleich oder verschieden sind und für Fluor, Chlor, Wasserstoff oder $C_1$–$C_4$-Alkylgruppen stehen, wobei die Wasserstoffatome der Alkylgruppe(n) gegebenenfalls vollständig oder partiell durch Fluor und/oder Chlor substituiert sein können, und mindestens einer der Reste $R_1$ bis $R_4$ für Fluor und mindestens einer der Reste $R_2$ und $R_4$ für Chlor steht, in Gegenwart von einem tertiären Amin ohne aktive Wasserstoffatome, Bariumoxid, Calciumcarbonat, Natriumcarbonat und/oder Kaliumcarbonat und eines Hydrierkatalysators, der aus Metallen und/oder Verbindungen von Elementen der 8. Nebengruppe des periodischen Systems der Elemente nach Mendelejew besteht oder diese enthält, und bei einem Wasserstoffdruck im Bereich von 1,2 bis 25 bar und Reaktionstemperaturen zwischen 20 und 100 °C hydriert.

Von den einzusetzenden Fluor und Chlor enthaltenden Pyrimidinen sind solche bevorzugt, bei denen in Formel (I) $R_1$, $R_2$, $R_3$ und $R_4$ gleich oder verschieden sind und für Fluor, Chlor, $CF_3$, $CF_2Cl$, $CCl_2F$, $CCl_3$, $CH_3$, $CHCl_2$, $CHF_2$ oder $CH_2F$ stehen, wobei mindestens einer der Reste $R_1$ bis $R_4$ für Fluor und $R_2$ und/oder $R_4$ für Chlor steht.

Besonders bevorzugt sind solche Fluor und Chlor enthaltende Pyrimidine, bei denen in Formel (I) $R_1$, $R_2$ und $R_3$ gleich oder verschieden sind und für Fluor, Chlor oder $CF_3$ stehen und $R_4$ für Fluor oder Chlor steht, wobei mindestens einer der Reste $R_1$ bis $R_4$ für Fluor, mindestens einer der Reste $R_2$ und $R_4$ für Chlor und $R_4$ nur dann für Fluor steht, wenn $R_2$ für Chlor steht.

Die einzusetzenden Fluor und Chlor enthaltenden Pyrimidine sind gut zugänglich. Sie sind entweder bekannt oder können auf analoge Weise wie die bekannten Fluor und Chlor enthaltenden Pyrimidine hergestellt werden, z.B. durch partielle Fluorierung von Chlor enthaltenden Pyrimidinen (siehe J. Fluorine Chem. 21, 495–513 (1982)) oder durch selektive Rückchlorierung von Fluor enthaltenden Pyrimidinen (siehe DE-A-3 328 154).

Das erfindungsgemässe Verfahren wird in Gegenwart von einem tertiären Amin ohne aktive Wasserstoffatome, Bariumoxid, Kalziumcarbonat, Natriumcarbonat und/oder Kaliumcarbonat durchgeführt. Tertiäre Amine mit aktiven Wasserstoffatomen können in unerwünschter Weise mit Halogenatomen am Pyrimidinring unter Bildung von Pyrimidinaminen reagieren. Als tertiäre Amine sind $C_1$–$C_4$-Alkylamine und Pyridin bevorzugt. Besonders bevorzugt wird Triethylamin eingesetzt.

Der Chlorwasserstoff-Akzeptor, d.h. das tertiäre Amin ohne aktive Wasserstoffatome und/oder die genannten basisch reagierenden anorganischen Verbindungen, wird vorzugsweise in stöchiometrischer Menge eingesetzt oder im geringen Überschuss, beispielsweise in einem Überschuss von bis zu 10 Mol-% bezogen auf die stöchiometrisch erforderliche Menge. Besonders bevorzugt wird die stöchiometrisch erforderliche Menge eingesetzt.

Sofern das eingesetzte Fluor und Chlor enthaltende Pyrimidin mehr als ein durch Hydrierung abspaltbares Chloratom aufweist, können diese Chloratome selektiv nacheinander abgespalten werden. Die Anzahl der abzuspaltenden Chloratome kann durch die Menge des eingesetzten Chlorwasserstoff-Akzeptors beeinflusst werden. Wird in das erfindungsgemässe Verfahren z.B. ein Chlorpyrimidin mit zwei durch Hydrierung abspaltbaren Chloratomen und pro Mol eines solchen Chlorpyrimidins ein Mol eines Chlorwasserstoff-Akzeptors eingesetzt, so wird selektiv nur ein Chloratom abgespalten. Wenn in diesem Fall pro Mol Chlorpyrimidin zwei Mole eines Chlorwasserstoff-Akzeptors eingesetzt werden, werden selektiv beide Chloratome abgespalten.

Das erfindungsgemässe Verfahren wird in Gegenwart von Hydrierkatalysatoren durchgeführt, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin und Nickel und deren Verbindungen bevorzugt. Bei den Metallverbindungen kann es sich beispielsweise um Oxide, Hydroxide und/oder Oxidhydrate handeln. Zusätzlich können die Metalle Kupfer, Vanadin, Molybdän, Chrom und/oder Mangan, sowie Verbindungen dieser Metalle zugegen sein.

Die Hydrierkatalysatoren können ausschliesslich oder überwiegend aus Wasserstoff-übertragenden Substanzen bestehen, diese können aber auch auf Trägermaterialien aufgebracht sein. Als Trägermaterialien für die Wasserstoff-übertragenden Substanzen kommen beispielsweise in Frage: anorganische Materialien wie Kieselgur, Kieselsäure, Aluminiumoxide, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten wie Seide, Polyamide, Polystyrole, Zellstoff oder Polyurethane. Bevorzugt sind anorganische Trägermaterialien. Das Trägermaterial kann beispielsweise in Form von Kugeln, Strängen, Fäden, Zylindern, Polygonen oder in Pulverform vorliegen.

Derartige Trägerkatalysatoren können im allgemeinen 0,5 bis 50 Gew.-%, vorzugsweise 1 bis 10 Gew.-% der wasserstoffübertragenden Substanz, bezogen auf die Gesamtmasse des Trägerkatalysators, enthalten. Die wasserstoffübertragende Substanz kann dabei homogen im Trägermaterial verteilt sein, bevorzugt sind jedoch Katalysatoren, in deren äusserer Schicht oder auf deren Oberfläche die wasserstoffübertragende Substanz abgelagert ist. Die Herstellung und die Formgebung von Katalysatoren, die im erfindungsgemässen Verfahren Verwendung finden können, kann in bekannter Weise erfolgen (siehe beispielsweise Houben-Weyl, Methoden der organischen Chemie, Band IV, 1c, Teil I, S. 16–26, Georg Thieme Verlag, Stuttgart 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid.

Bevorzugte Hydrierkatalysatoren, die ausschliesslich oder überwiegend aus wasserstoffübertragender Substanz bestehen, sind beispielsweise oxidische Katalysatoren wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit z.B. Hydrazin, Formaldehyd, Wasserstoff oder elektropositiveren Metallen herstellbare Schwarzkatalysatoren, wie Palladium-Schwarz, Platin-Schwarz und Rhodium-Schwarz.

Besonders bevorzugte Katalysatoren für das erfindungsgemässe Verfahren sind Palladium auf Kohle, Palladium auf Aluminiumoxid, Palladium auf Kieselsäure und Palladium auf Calciumcarbonat.

Es können auch Skelettkatalysatoren vom Raney-Typ eingesetzt werden, wobei Raney-Nickel bevorzugt ist.

Der Hydrierkatalysator kann in das erfindungsgemässe Verfahren beispielsweise in einer solchen Menge eingesetzt werden, dass 0,05 bis 2,5 Gew.-% wasserstoffübertragende Substanz, bezogen auf das Gesamtgewicht des Reaktionsgemisches, vorliegen. Vorzugsweise beträgt die Menge 0,1 bis 1 Gew.-%.

Zur Durchführung des erfindungsgemässen Verfahrens können auch Gemische aus zwei oder mehreren der genannten Hydrierkatalysatoren verwendet werden.

Die katalytische Aktivität der Hydrierkatalysatoren bleibt bei der Durchführung des erfindungsgemässen Verfahrens im allgemeinen weitgehend erhalten, sodass diese bei diskontinuierlicher Arbeitsweise wiederholt und bei kontinuierlicher Arbeitsweise für längere Zeit eingesetzt werden können.

Im allgemeinen ist es vorteilhaft, das erfindungsgemässe Verfahren in Gegenwart eines Lösungsmittels durchzuführen.

Als Lösungsmittel kommen beispielsweise inerte organische Lösungsmittel in Frage. Geeignet sind beispielsweise Alkohole wie Methanol, Ethanol, Ethylenglykol und Diethylenglykol, Ether wie Dioxan, Tetrahydrofuran, Ethylenglykolmonomethylether. Ethylenglykolmonoethylether, Ethylenglykoldimethylether, Diethylenglykolmonomethylether und Diethylenglykoldimethylether, gesättigte Kohlenwasserstoffe wie Cyclohexan oder Ester wie Essigsäureethylester. Aufgrund ihrer Lösungseigenschaften und ihrer niederen Siedepunkte sind Methanol, Ethanol und Essigsäureethylester besonders bevorzugte Lösungsmittel.

Das erfindungsgemässe Verfahren kann in Reaktionsapparaturen durchgeführt werden, die für Druckhydrierungen geeignet sind. Geeignete Materialien für die Reaktionsapparaturen sind beispielsweise Glas, Emaille, Stahl oder Edelstahl.

Der Wasserstoffdruck, unter dem die Hydrierung durchgeführt wird, liegt im Bereich von 1,2 bis 25 bar. Bevorzugt ist ein Wasserstoffdruck von 1,5 bis 15 bar, besonders bevorzugt einer von 2 bis 10 bar.

Die Reaktionstemperatur liegt zwischen 20 und 100 °C. Bevorzugt sind Temperaturen zwischen 20 und 90 °C, besonders bevorzugt sind solche zwischen 30 und 80 °C.

Die für das erfindungsgemässe Verfahren erforderliche Reaktionszeit ist abhängig von der Reaktionsgeschwindigkeit, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden.

Das erfindungsgemässe Verfahren kann beispielsweise in der einfachsten Ausführungsform diskontinuierlich in folgender Weise durchgeführt werden: Ein mit einer Rühr- oder Mischeinrichtung versehener, temperierbarer Autoklav wird in geeigneter Weise mit zu hydrierendem Fluor und Chlor enthaltendem Pyrimidin, dem Hydrierkatalysator, einem Lösungsmittel und einem der genannten Chlorwasserstoff-Akzeptoren beschickt. Danach wird Wasserstoff bis zum gewünschten Druck aufgedrückt und das Gemisch unter intensiver Durchmischung auf die gewählte Reaktionstemperatur erhitzt. Der Reaktionsverlauf lässt sich leicht durch Messung des Wasserstoffverbrauchs, der durch weitere Wasserstoffzufuhr ausgeglichen wird, verfolgen. Die Hydrierung ist beendet, wenn kein Wasserstoff mehr verbraucht wird und die verbrauchte Wasserstoffmenge etwa der theoretisch erforderlichen Wasserstoffmenge entspricht.

Die Aufarbeitung des nach der Hydrierung vorliegenden Gemisches kann beispielsweise so erfolgen, dass man nach Beendigung der Hydrierung abkühlt, entspannt und das feste Amin-Hydrochlorid oder die vorliegenden anorganischen Salze, sowie den Katalysator abfiltriert, mit dem verwendeten Lösungsmittel nachwäscht und anschliessend das Lösungsmittel bei vermindertem Druck oder bei Normaldruck entfernt. Das zurückbleibende Rohprodukt kann ebenfalls durch eine Destillation unter vermindertem Druck oder unter Normaldruck oder, bei kristallinem Rohprodukt, durch Umkristallisation weiter gereinigt werden. Bei Verwendung von hochsiedenden Lösungsmitteln kann auch zunächst das hydrierte Pyrimidin-Derivat abdestilliert werden.

Die mit den erfindungsgemässen Verfahren zugänglichen teilweise bekannten, zum grössten Teil jedoch neuen Produkte der Formel (II)

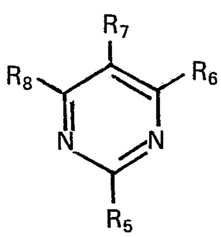

(II)

in der

$R_5$, $R_6$, $R_7$ und $R_8$ gleich oder verschieden sind und für Fluor, Chlor, Wasserstoff oder $C_1$–$C_4$-Alkylgruppen stehen, wobei die Wasserstoffatome der Alkylgruppe(n) gegebenenfalls vollständig oder partiell durch Fluor und/oder Chlor substituiert sein können, und wobei mindestens einer der Reste $R_5$ bis $R_8$ für Fluor und mindestens einer der Reste $R_6$ und $R_8$ für Wasserstoff steht, sind als solche oder ergeben nach weiterer Umsetzung Zwischenprodukte für Herbizide, Wachstumsregulatoren und Pharmazeutika.

Beispielsweise kann man Produkte der Formel (II), bei denen $R_5$ und $R_8$ gleich oder verschieden sind und für Fluor und/oder Chlor stehen, $R_6$ für Wasserstoff steht und $R_7$ die oben angegebene Bedeutung hat, durch alkalische oder saure Verseifung in die entsprechenden Pyrimidindione (Urazile) überführen, die Zwischenprodukte für die Herstellung von Herbiziden und Wachstumsregulatoren sind.

So ist beispielsweise 5-Fluor-2,4(1H, 3H)-pyrimidindion (= 5-Fluoruracil) zugänglich, das ein bekanntes Cancerostatikum ist und auch als Ausgangsprodukt zur Synthese von Tumorwachstuminhibierenden Nucleosid-Derivaten verwendet wird (siehe z.B. Biochemical Aspects of Fluorine Chemistry, Edited by R. Filler and Y. Kobayaski, Kodanska Ltd. Tokyo and Elsevier Biomedical Press Amsterdam-New-York-Oxford (1982) und die dort zitierte Literatur).

Die vorstehend erwähnten neuen Pyrimidine sind durch die Formel (III)

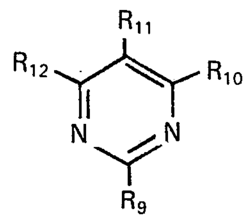

(III)

gekennzeichnet, in der

$R_9$, $R_{10}$, $R_{11}$ und $R_{12}$ gleich oder verschieden sind und für Fluor, Chlor, Wasserstoff oder $C_1$–$C_4$-Alkylgruppen stehen, wobei die Wasserstoffatome der Alkylgruppe(n) gegebenenfalls vollständig oder partiell durch Fluor und/oder Chlor substituiert sein können, wobei mindestens einer der Reste $R_9$ bis $R_{12}$ für Fluor und mindestens einer der Reste $R_{10}$ und $R_{12}$ für Wasserstoff steht und die kombinierten Definitionen

$R_9$ und $R_{11}$ = F, $R_{10}$ und $R_{12}$ = H,
$R_9$ = Cl, $R_{10}$ und $R_{12}$ = H, $R_{11}$ = F,
$R_9$ und $R_{12}$ = Cl, $R_{10}$ = H, $R_{11}$ = F und
$R_9$, $R_{11}$ und $R_{12}$ = F, $R_{10}$ = H,

ausgenommen sind.

Bevorzugte Pyrimidine der Formel (III) sind solche, bei denen $R_9$, $R_{10}$, $R_{11}$ und $R_{12}$ gleich oder verschieden sind und für Fluor, Chlor, Wasserstoff, $CF_3$, $CF_2Cl$, $CFCl_2$, $CCl_3$, $CH_3$, $CHCl_2$, $CHF_2$ oder $CH_2F$ stehen, wobei mindestens einer der Reste $R_9$ bis $R_{12}$ für Fluor und $R_{10}$ und/oder $R_{12}$ für Wasserstoff steht und die bei der allgemeinen Erläuterung der Formel (III) ausgenommenen Kombinationen der Reste $R_9$ bis $R_{12}$ auch hier ausgenommen sind.

Weitere bevorzugte Pyrimidine der Formel (III) sind solche, bei denen $R_9$ und $R_{10}$ gleich oder verschieden sind und für Fluor, Chlor, Wasserstoff, Methyl oder eine beliebig mit Fluor und/oder Chlor substituierte Methylgruppe, $R_{11}$ für Fluor und $R_{12}$ für Wasserstoff stehen, wobei die bei der allgemeinen Erläuterung der Formel (III) ausgenommenen Kombinationen der Reste $R_9$ bis $R_{12}$ auch hier ausgenommen sind.

Weitere bevorzugte Pyrimidine der Formel (III) sind solche, bei denen $R_9$ und $R_{11}$ gleich oder ver-

schieden sind und für Fluor, Chlor, Wasserstoff, Methyl oder eine beliebig mit Fluor und/oder Chlor substituierte Methylgruppe, $R_{10}$ für Wasserstoff oder Fluor und $R_{12}$ für Wasserstoff stehen, wobei die bei der allgemeinen Erläuterung der Formel (III) ausgenommenen Kombinationen der Reste $R_9$ bis $R_{12}$ auch hier ausgenommen sind und mindestens einer der Reste $R_9$ bis $R_{12}$ für Fluor steht.

Besonders bevorzugte Pyrimidine der Formel (III) sind solche, bei denen $R_9$ für Fluor, Chlor, Wasserstoff, Methyl oder eine beliebig mit Fluor und/oder Chlor substituierte Methylgruppe, $R_{10}$ für Wasserstoff, $R_{11}$ für Fluor und $R_{12}$ für Wasserstoff, Fluor oder Chlor stehen, wobei die bei der allgemeinen Erläuterung der Formel (III) ausgenommenen Kombinationen der Reste $R_9$ bis $R_{12}$ auch hier ausgenommen sind.

Weitere besonders bevorzugte Pyrimidine der Formel (III) sind solche, bei denen $R_{10}$ für Fluor, Chlor, Wasserstoff, Methyl oder eine beliebig mit Fluor und/oder Chlor substituierte Methylgruppe, $R_9$ für Fluor oder Chlor, $R_{11}$ für Fluor und $R_{12}$ für Wasserstoff stehen, wobei die bei der allgemeinen Erläuterung der Formel (III) ausgenommenen kombinierten Definitionen der Reste $R_9$ bis $R_{12}$ auch hier ausgenommen sind.

Weitere besonders bevorzugte Pyrimidine der Formel (III) sind solche, bei denen $R_{11}$ für Fluor, Chlor, Wasserstoff, Methyl oder eine beliebig mit Fluor und/oder Chlor substituierte Methylgruppe, $R_{10}$ für Wasserstoff, $R_9$ für Fluor oder Chlor und $R_{12}$ für Wasserstoff, Fluor oder Chlor stehen, wobei die bei der allgemeinen Erläuterung der Formel (III) ausgenommenen Kombinationen der Reste $R_9$ bis $R_{12}$ auch hier ausgenommen sind und mindestens einer der Reste $R_9$ bis $R_{12}$ für Fluor steht.

Ein ganz besonders bevorzugtes Pyrimidin der Formel (III) ist das, bei dem $R_9$ und $R_{11}$ für Fluor, $R_{10}$ für Wasserstoff und $R_{12}$ für Chlor stehen.

Die Herstellung und die Verwendbarkeit der neuen Pyrimidine der Formel (III) ist bereits vorstehend beschrieben worden.

Es ist ausgesprochen überraschend, dass es mit dem erfindungsgemässen Verfahren gelingt, Pyrimidine, die bisher nur auf sehr unzulängliche Weise zugänglich waren, auf einfache Weise und in guten Ausbeuten herzustellen. Ausserdem ist es überraschend, dass mit dem erfindungsgemässen Verfahren neue Pyrimidine zugänglich werden, die wertvolle Zwischenprodukte darstellen. Es konnte keinesfalls erwartet werden, dass die erfindungsgemässe Hydrierung zum Erfolg führt, denn sowohl der Pyrimidin-Kern, als auch an den Pyrimidinkern gebundene Substituenten, insbesondere weitere Fluor- und/oder Chloratome, sind der Hydrierung zugänglich, werden unerwarteterweise jedoch nicht angegriffen.

Die folgenden Beispiele erläutern das erfindungsgemässe Verfahren und die erfindungsgemässen neuen Pyrimidine, ohne die vorliegende Erfindung darauf zu beschränken.

## Beispiele

### Beispiel 1

185 g (1 Mol) 2,5-Difluor-4,6-dichlorpyrimidin wurden in 1800 ml Essigsäureethylester unter Zusatz von 110 g Triethylamin und 15 g Palladium auf Kohle (5 gew.-%ig) bei 30 °C und einem Wasserstoffdruck von 3,5 bar in einem Edelstahl-Rührautoklaven während 95 Minuten hydriert. Anschliessend wurden die festen Anteile des Reaktionsgemisches abfiltriert, der Rückstand mit Essigsäureethylester gewaschen und das mit der Waschflüssigkeit vereinigte Filtrat bei Normaldruck über eine 30 cm-Füllkörperkolonne destilliert. Nachdem das Lösungsmittel abdestilliert war, wurden 106 g 2,5-Difluor-4-chlorpyrimidin mit einem Siedepunkt von 145 bis 146 °C erhalten. Die Ausbeute betrug demnach 70,5% der Theorie. Nach gaschromatographischer Analyse wies das isolierte Reaktionsprodukt eine Reinheit von 94,7% auf.

### Beispiele 2 bis 10

Es wurde verfahren wie in Beispiel 1 beschrieben, jedoch wurden andere Ausgangsprodukte eingesetzt. Die Ausgangsprodukte, die Reaktionsprodukte und sonstige Daten sind in Tabelle 1 angegeben.

Tabelle 1

| Beispiel | Ausgangsprodukt 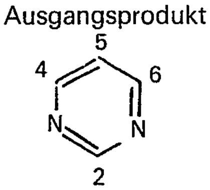 | Reaktionsprodukt 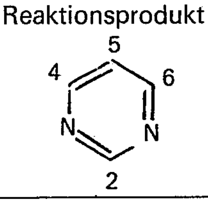 | Ausbeute (% der Theorie) | Siedepunkt (°C/mbar) | Reinheit (%) |
|---|---|---|---|---|---|
| 2 | 2,4,6-Trichlor-5-fluor | 2,4-Dichlor-5-fluor | 68 | 88–91/30 | 94,1 |
| 3 | 2,4,5-Trifluor-6-chlor | 2,4,5-Trifluor | 87 | 92–93/1013 | 98,4 |
| 4 | 2,5-Difluor-4-chlor-6-trifluormethyl | 2,5-Difluor-6-trifluormethyl | 88 | 115–116/1013 | 96,6 |
| 5 | 2,4-Dichlor-5-fluor-6-trifluormethyl | 2-Chlor-5-fluor-6-trifluormethyl | 75 | 138–140/300 | 93,1 |

Tabelle 1 (Fortsetzung)

| Beispiel | Ausgangsprodukt 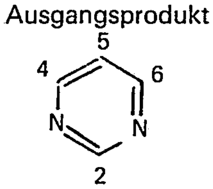 | Reaktionsprodukt 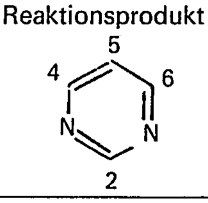 | Ausbeute (% der Theorie) | Siedepunkt (°C/mbar) | Reinheit (%) |
|---|---|---|---|---|---|
| 6 | 2-Trifluormethyl-4-chlor-5,6-difluor | 2-Trifluormethyl-5,6-difluor | 86,6 | 113–114/1013 | 95,2 |
| 7 | 2-Trifluormethyl-4,6-dichlor-5-fluor | 2-Trifluormethyl-4-chlor-5-fluor | 95,5 | 123–125/1013 | 98,6 |
| 8 | 2-Chlordifluormethyl-4,5-dichlor-6-fluor | 2-Chlordifluormethyl-5-chlor-6-fluor | 73 | 34–35/80 | 97,0 |
| 9 | 2-Methyl-5,6-dichlor-4-fluor | 2-Methyl-5-chlor-4-fluor | 76 | *) | 96,8 |
| 10 | 2-Fluor-4,5-dichlor-6-methyl | 2-Fluor-5-chlor-6-methyl | 93 | 58–60/20 | 95,7 |

*) Schmelzpunkt 220–222 °C, isoliert durch Filtrieren und Umkristallisation aus Essigsäureethylester.

Beispiel 11

50 g (0,27 Mol) 2,5-Difluor-4,6-dichlorpyrimidin wurden in 500 ml Essigsäureethylester unter Zusatz von 60 g Triethylamin und 7 g Palladium auf Kohle (5 gew.-%ig) bei 50 °C und einem Wasserstoffdruck von 4 bar in einer Glasapparatur während 3,5 Stunden hydriert. Anschliessend wurden die festen Anteile des Reaktionsgemisches abfiltriert, der Rückstand mit Essigsäureethylester gewaschen und das mit der Waschflüssigkeit vereinigte Filtrat bei Normaldruck über eine 30 cm-Füllkörperkolonne destilliert. Nachdem das Lösungsmittel abdestilliert war, wurden 25,7 g 2,5-Difluorpyrimidin mit einem Siedepunkt von 116 bis 117 °C erhalten. Die Ausbeute betrug demnach 82,2% der Theorie. Nach gaschromatographischer Analyse wies das isolierte Produkt eine Reinheit von 98,3% auf.

Beispiele 12 bis 14

Es wurde verfahren wie bei Beispiel 11 beschrieben, jedoch wurden andere Ausgangsprodukte eingesetzt. Die Ausgangsprodukte, die Reaktionsprodukte und sonstige Daten sind in Tabelle 2 angegeben.

Tabelle 2

| Beispiel | Ausgangsprodukt 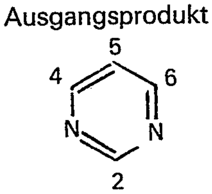 | Reaktionsprodukt 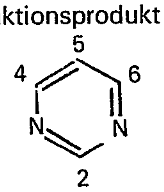 | Ausbeute (% der Theorie) | Siedepunkt (°C/mbar) | Reinheit (%) |
|---|---|---|---|---|---|
| 12 | 2,4,6-Trichlor-5-fluor | 2-Chlor-5-fluor | 78 | 92–94/100 | 96,9 |
| 13 | 2-Trifluormethyl-4,6-dichlor-5-fluor | 2-Trifluormethyl-5-fluor | 92 | 130–132/1013 | 99,6 |
| 14 | 2-Trichlormethyl-4,6-dichlor-5-fluor | 2-Trichlormethyl-5-fluor | 63 | 120–122/30 | 92,7 |

Die Charakterisierung der Reaktionsprodukte aus den Beispielen 12 bis 14 erfolgte zusätzlich mittels $^{19}F$- und $^{1}H$-NMR-Spektroskopie, sowie mittels Massenspektrometer.

Beispiel 15

(Verwendung eines erfindungsgemäss hergestellten Stoffes)

150,5 g (1 Mol) des gemäss Beispiel 1 erhaltenen 2,5-Difluor-4-chlorpyrimidins wurden in 1,5 l Wasser aufgenommen und unter Rühren auf 80 °C (Innentemperatur) erhitzt. Bereits während des Aufheizens wurde damit begonnen, 200 g 45 Gew.-%ige wässrige Natronlauge zuzutropfen. Nach beendeter Zugabe wurde 4 Stunden bei 80 °C nachgerührt. Nach dem Erkalten wurde mit

konzentrierter Salzsäure neutralisiert und der ausgefallene Feststoff abgesaugt. Dieser Feststoff wurde mit 500 ml Wasser digeriert, erneut abgesaugt und bis zur Wasserfreiheit bei 50 °C im Vakuum getrocknet. Es wurden 121 g (93% der Theorie) 5-Fluor-2,4-(1H, 3H)-pyrimidindion (= 5-Fluoruracil) mit einem Schmelzpunkt von 280 bis 282 °C (Zersetzung) erhalten.

## Patentansprüche

1. Verfahren zum teilweisen oder vollständigen Entchlorieren von Fluor und Chlor enthaltenden Pyrimidinen, dadurch gekennzeichnet, dass man Fluor und Chlor enthaltende Pyrimidine der Formel (I)

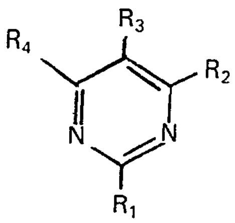

(I),

in der

$R_1$, $R_2$, $R_3$ und $R_4$ gleich oder verschieden sind und für Fluor, Chlor, Wasserstoff oder $C_1$–$C_4$-Alkylgruppen stehen, wobei die Wasserstoffatome der Alkylgruppe(n) gegebenenfalls vollständig oder partiell durch Fluor und/oder Chlor substituiert sein können, und wobei mindestens einer der Reste $R_1$ bis $R_4$ für Fluor und mindestens einer der Reste $R_2$ und $R_4$ für Chlor steht, in Gegenwart von einem tertiären Amin ohne aktive Wasserstoffatome, Bariumoxid, Calciumcarbonat, Natriumcarbonat und/oder Kaliumcarbonat und eines Hydrierkatalysators, der aus Metallen und/oder Verbindungen von Elementen der 8. Nebengruppe des periodischen Systems der Elemente nach Mendelejew besteht oder diese enthält und bei einem Wasserstoffdruck im Bereich von 1,2 bis 25 bar und Reaktionstemperaturen zwischen 20 und 100 °C hydriert.

2. Verfahren nach Anspruch 1, dadurch gekennzeichnet, dass man Fluor und Chlor enthaltende Pyrimidine der angegebenen Formel hydriert, bei denen $R_1$, $R_2$, $R_3$ und $R_4$ gleich oder verschieden sind und für Fluor, Chlor, $CF_3$, $CF_2Cl$, $CCl_2F$, $CCl_3$, $CH_3$, $CHCl_2$, $CHF_2$ oder $CH_2F$ stehen, wobei mindestens einer der Reste $R_1$ bis $R_4$ für Fluor und $R_2$ und/oder $R_4$ für Chlor steht.

3. Verfahren nach Ansprüchen 1 und 2, dadurch gekennzeichnet, dass man Fluor und Chlor enthaltende Pyrimidine der angegebenen Formel hydriert, bei denen $R_1$, $R_2$ und $R_3$ gleich oder verschieden sind und für Fluor, Chlor oder $CF_3$ stehen und $R_4$ für Fluor oder Chlor steht, wobei mindestens einer der Reste $R_1$ bis $R_4$ für Fluor, mindestens einer der Reste $R_2$ und $R_4$ für Chlor und $R_4$ nur dann für Fluor steht, wenn $R_2$ für Chlor steht.

4. Neue Pyrimidine der Formel (III)

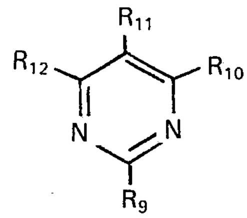

(III),

in der

$R_9$, $R_{10}$, $R_{11}$ und $R_{12}$ gleich oder verschieden sind und für Fluor, Chlor, Wasserstoff oder $C_1$–$C_4$-Alkylgruppen stehen, wobei die Wasserstoffatome der Alkylgruppe(n) gegebenenfalls vollständig oder partiell durch Fluor und/oder Chlor substituiert sein können, wobei mindestens einer der Reste $R_9$ bis $R_{12}$ für Fluor und mindestens einer der Reste $R_{10}$ und $R_{12}$ für Wasserstoff steht und die kombinierten Definitionen

$R_9$ und $R_{11}$ = F, $R_{10}$ und $R_{12}$ = H,
$R_9$ = Cl, $R_{10}$ und $R_{12}$ = H, $R_{11}$ = F,
$R_9$ und $R_{12}$ = Cl, $R_{10}$ = H, $R_{11}$ = F und
$R_9$, $R_{11}$ und $R_{12}$ = F, $R_{10}$ =H,

ausgenommen sind.

5. Neue Pyrimidine gemäss der in Anspruch 4 angegebenen Formel, dadurch gekennzeichnet, dass in der Formel

$R_9$, $R_{10}$, $R_{11}$ und $R_{12}$ gleich oder verschieden sind und für Fluor, Chlor, Wasserstoff, $CF_3$, $CF_2Cl$, $CFCl_2$, $CCl_3$, $CH_3$, $CHCl_2$, $CHF_2$ oder $CH_2F$ stehen, wobei mindestens einer der Reste $R_9$ bis $R_{12}$ für Fluor und $R_{10}$ und/oder $R_{12}$ für Wasserstoff steht und die kombinierten Definitionen

$R_9$ und $R_{11}$ = F, $R_{10}$ und $R_{12}$ = H
$R_9$ = Cl, $R_{10}$ und $R_{12}$ = H, $R_{11}$ = F,
$R_9$ und $R_{12}$ = Cl, $R_{10}$ = H, $R_{11}$ = F und
$R_9$, $R_{11}$ und $R_{12}$ = F, $R_{10}$ = H,

ausgenommen sind.

6. Neues Pyrimidin gemäss der in Anspruch 4 angegebenen Formel, dadurch gekennzeichnet, dass $R_9$ und $R_{11}$ für Fluor, $R_{10}$ für Wasserstoff und $R_{12}$ für Chlor stehen.

## Revendications

1. Procédé pour la déchloration partielle ou totale de pyrimidines contenant du fluor et du chlore, caractérisé en ce que l'on hydrogène des pyrimidines contenant du fluor et du chlore et répondant à la formule I

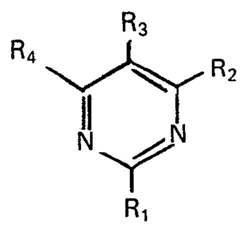

(I)

dans laquelle

$R_1$, $R_2$, $R_3$ et $R_4$, ayant des significations identiques ou différentes, représentent chacun le fluor, le chlore, l'hydrogène ou un groupe alkyle en $C_1$–$C_4$, les atomes d'hydrogène du ou des groupes alkyle pouvant être remplacés éventuellement en totalité ou en partie par du fluor et/ou du chlore, l'un au moins des symboles $R_1$ à $R_4$ représentant

le fluor et l'un au moins des symboles $R_2$ et $R_4$ le chlore, en présence d'une amine tertiaire ne portant pas d'atome d'hydrogène actif, d'oxyde de baryum, de carbonate de calcium, de carbonate de sodium et/ou de carbonate de potassium et d'un catalyseur d'hydrogénation consistant en totalité ou en partie en métaux et/ou composés des éléments du 8e sous-groupe de la Classification Périodique des Eléments selon Mendeleieff, sous une pression d'hydrogène dans l'intervalle de 1,2 à 25 bar et à des températures de réaction de 20 à 100 °C.

2. Procédé selon la revendication 1, caractérisé en ce que l'on hydrogène des pyrimidines contenant du fluor et du chlore et répondant à la formule indiquée dans laquelle $R_1$, $R_2$, $R_3$ et $R_4$, ayant des significations identiques ou différentes, représentent chacun le fluor, le chlore, un groupe $CF_3$, $CF_2Cl$, $CCl_2F$, $CCl_3$, $CH_3$, $CHCl_2$, $CHF_2$ ou $CH_2F$, l'un au moins des symboles $R_1$ à $R_4$ représentant le fluor et $R_2$ et/ou $R_4$ le chlore.

3. Procédé selon les revendications 1 et 2, caractérisé en ce que l'on hydrogène des pyrimidines contenant du fluor et du chlore et répondant à la formule indiquée dans laquelle $R_1$, $R_2$, $R_3$, ayant des significations identiques ou différentes, représentent chacun le fluor, le chlore ou un groupe $CF_3$ et $R_4$ représente le fluor ou le chlore, l'un au moins des symboles $R_1$ à $R_4$ représentant le fluor, l'un au moins des symboles $R_2$ et $R_4$ représentant le chlore et $R_4$ ne pouvant représenter le fluor que lorsque $R_2$ représente le chlore.

4. Nouvelles pyrimidines de formule III

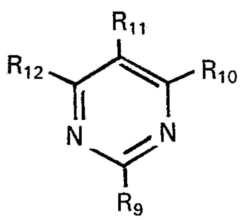

(III)

dans laquelle

$R_9$, $R_{10}$, $R_{11}$ et $R_{12}$, ayant des significations identiques ou différentes, représentent chacun le fluor, le chlore, l'hydrogène ou un groupe alkyle en $C_1$–$C_4$, les atomes d'hydrogène du ou des groupes alkyle pouvant éventuellement être remplacés en totalité ou en partie par le fluor et/ou le chlore, l'un au moins des symboles $R_9$ à $R_{12}$ représentant le fluor et l'un au moins des symboles $R_{10}$ et $R_{12}$ l'hydrogène, les combinaisons des définitions suivantes:

$R_9$ et $R_{11}$ = F, $R_{10}$ et $R_{12}$ = H,
$R_9$ = Cl, $R_{10}$ et $R_{12}$ = H, $R_{11}$ = F,
$R_9$ et $R_{12}$ = Cl, $R_{10}$ = H, $R_{11}$ = F, et
$R_9$, $R_{11}$ et $R_{12}$ = F, $R_{10}$ = H,

étant exceptées.

5. Nouvelles pyrimidines répondant à la formule indiquée dans la revendication 4 et caractérisées en ce que, dans cette formule:

$R_9$, $R_{10}$, $R_{11}$ et $R_{12}$, ayant des significations identiques ou différentes, représentent chacun le fluor, le chlore, l'hydrogène, un groupe $CF_3$, $CF_2Cl$, $CFCl_2$, $CCl_3$, $CH_3$, $CHCl_2$, $CHF_2$ ou $CH_2F$, l'un au moins des symboles $R_9$ à $R_{12}$ représentant le fluor et $R_{10}$ et/ou $R_{12}$ l'hydrogène, à l'exception des définitions combinées suivantes:

$R_9$ et $R_{11}$ = F, $R_{10}$ et $R_{12}$ = H,
$R_9$ = Cl, $R_{10}$ et $R_{12}$ = H, $R_{11}$ = F,
$R_9$ et $R_{12}$ = Cl, $R_{10}$ = H, $R_{11}$ = F, et
$R_9$, $R_{11}$ et $R_{12}$ = F, $R_{10}$ = H.

6. Nouvelle pyrimidine répondant à la formule indiquée dans la revendication 4 et caractérisée en ce que $R_9$ et $R_{11}$ représentent le fluor, $R_{10}$ l'hydrogène et $R_{12}$ le chlore.

**Claims**

1. Process for the partial or complete dechlorination of pyrimidines containing fluorine and chlorine, characterized in that pyrimidines containing fluorine and chlorine, of the formula (I)

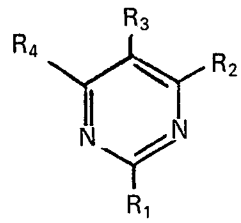

(I)

in which

$R_1$, $R_2$, $R_3$ and $R_4$ are identical or different and represent fluorine, chlorine, hydrogen or $C_1$–$C_4$-alkyl groups, where the hydrogen atoms of the alkyl group(s) can optionally be completely or partially substituted by fluorine and/or chlorine, and where at least one of the radicals $R_1$ to $R_4$ represents fluorine and at least one of the radicals $R_2$ and $R_4$ represents chlorine, are hydrogenated in the presence of a tertiary amine without active hydrogen atoms, barium oxide, calcium carbonate, sodium carbonate and/or potassium carbonate and of a hydrogenation catalyst which consists of metals and/or compounds of elements of the 8th subgroup of Mendeleev's periodic table of the elements, or which contains these, at a hydrogen pressure in a range from 1.2 to 25 bar and at reaction temperatures between 20 and 100 °C.

2. Process according to Claim 1, characterized in that pyrimidines containing fluorine and chlorine, of the formula given, in which $R_1$, $R_2$, $R_3$ and $R_4$ are identical or different and represent fluorine, chlorine, $CF_3$, $CF_2Cl$, $CCl_2F$, $CCl_3$, $CH_3$, $CHCl_2$, $CHF_2$ or $CH_2F$, where at least one of the radicals $R_1$ to $R_4$ represents fluorine and $R_2$ and/or $R_4$ represent chlorine, are hydrogenated.

3. Process according to Claims 1 and 2, characterized in that pyrimidines containing fluorine and chlorine of the formula given, in which $R_1$, $R_2$ and $R_3$ are identical or different and represent fluorine, chlorine or $CF_3$, and $R_4$ represents fluorine or chlorine, where at least one of the radicals $R_1$ to $R_4$ represents fluorine, at least one of the radicals $R_2$ and $R_4$ represents chlorine, and $R_4$ only represents fluorine when $R_2$ represents chlorine, are hydrogenated.

4. New pyrimidines of the formula (III)

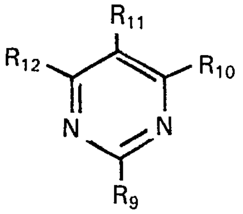

(III)

in which

$R_9$, $R_{10}$, $R_{11}$ and $R_{12}$ are identical or different and represent fluorine, chlorine, hydrogen or $C_1$–$C_4$-alkyl group(s) can optionally be completely or partially substituted by fluorine and/or chlorine, where at least one of the radicals $R_9$ to $R_{12}$ represents fluorine and at least one of the radicals $R_{10}$ and $R_{12}$ represents hydrogen and the combined definitions

$R_9$ and $R_{11}$ = F, $R_{10}$ and $R_{12}$ = H,
$R_9$ = Cl, $R_{10}$ and $R_{12}$ = H, $R_{11}$ = F,
$R_9$ and $R_{12}$ = Cl, $R_{10}$ = H, $R_{11}$ = F and
$R_9$, $R_{11}$ and $R_{12}$ = F, $R_{10}$ = H,
are excluded.

5. New pyrimidines according to the formula given in Claim 4, characterized in that, in the formula,

$R_9$, $R_{10}$, $R_{11}$ and $R_{12}$ are identical or different and represent fluorine, chlorine, hydrogen, $CF_3$, $CF_2Cl$, $CFCl_2$, $CCl_3$, $CH_3$, $CHCl_2$, $CHF_2$ or $CH_2F$, were at least one of the radicals $R_9$ to $R_{12}$ represent fluorine and $R_{10}$ and/or $R_{12}$ represents hydrogen and the combined definitions

$R_9$ and $R_{11}$ = F, $R_{10}$ and $R_{12}$ = H,
$R_9$ = Cl, $R_{10}$ and $R_{12}$ = H, $R_{11}$ = F,
$R_9$ and $R_{12}$ = Cl, $R_{10}$ = H, $R_{11}$ = F and
$R_9$, $R_{11}$ and $R_{12}$ = F, $R_{10}$ = H,
are excluded.

6. New pyrimidine according to the formula given in Claim 4, characterized in that $R_9$ and $R_{11}$ represent fluorine, $R_{10}$ represents hydrogen and $R_{12}$ represents chlorine.